(19) Europäisches Patentamt
European Patent Office
Office européen des brevets

(11) Veröffentlichungsnummer: **0 095 569**
**B1**

(12) # EUROPÄISCHE PATENTSCHRIFT

(45) Veröffentlichungstag der Patentschrift: **28.01.87**

(51) Int. Cl.[4]: **C 07 D 237/14**

(21) Anmeldenummer: **83103159.6**

(22) Anmeldetag: **30.03.83**

(54) **Verfahren zur Herstellung von Phenylpyridazinverbindungen.**

(30) Priorität: **19.05.82 DE 3218976**

(43) Veröffentlichungstag der Anmeldung: **07.12.83 Patentblatt 83/49**

(45) Bekanntmachung des Hinweises auf die Patenterteilung: **28.01.87 Patentblatt 87/5**

(84) Benannte Vertragsstaaten: **AT BE CH DE FR GB IT LI LU NL SE**

(56) Entgegenhaltungen:
**AT - A - 320 657**
**AT - A - 326 137**

(73) Patentinhaber: **CHEMIE LINZ AKTIENGESELLSCHAFT, St. Peter-Strasse 25, A-4020 Linz (AT)**
(84) Benannte Vertragsstaaten: **BE CH FR GB IT LI LU NL SE AT**

(73) Patentinhaber: **Lentia Gesellschaft mit beschränkter Haftung, Arabellastrasse 4 Postfach 81 05 08, D-8000 München 81 (DE)**
(84) Benannte Vertragsstaaten: **DE**

(72) Erfinder: **Raninger, Franz, Dr., Schmidbergerweg 3, A-4490 St. Florian (AT)**
Erfinder: **Kloimstein, Engelbert, Schiferplatz 22, A-4070 Eferding (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von Phenylpyridazinverbindungen in wässrig-acetonischem Medium.

Es ist aus DE-OS 2 331 398 bekannt, dass 3-Phenyl-6-chlorpyridazin-thiocarbonate wertvolle herbizide Eigenschaften besitzen. Ebenso ist dort deren Herstellung durch Umsatz der Verbindung 3-Phenyl-4-hydroxy-6-chlorpyridazin oder von deren Salzen mit Alkylthiochlorformiaten beschrieben, wobei die Umsetzung gegebenenfalls unter Zusatz eines Säureacceptors, nämlich eines tertiären Amins stattfindet, auf dessen Beigabe vornehmlich dann verzichtet werden kann, wenn das 3-Phenyl-4-hydroxy-6-chlorpyridazin in Form seines Salzes mit tertiären Aminen eingesetzt wird. Die Umsetzung erfolgt vorzugsweise in Gegenwart inerter Lösungsmittel, wie aromatische Kohlenwasserstoffe, während andererseits empfohlen wird, für eine Abwesenheit von hydroxylgruppenenthaltenden Verbindungen, wie Alkoholen oder Wasser zu sorgen.

Dieses Verfahren lässt in der Praxis viele Wünsche offen, weil die als Säureacceptor dienenden tertiären Amine, gleichgültig, ob sie als solche oder in Form der Salze mit der Pyridazinverbindung eingesetzt wurden, während der Reaktion in Hydrochloride übergeführt werden, die entweder unter starker Abwasserbelastung verworfen oder in einem gesonderten Verfahren aufgearbeitet werden müssen, was das Gesamtverfahren wirtschaftlich stark belastet. Es wurde weiters festgestellt, dass geringe Mengen von tertiären Aminen, vor allem in Anwesenheit von Wasser, zersetzend auf Verbindungen der Formel I wirken. Auch die als Reaktionsmedium angegebenen inerten aromatischen Kohlenwasserstoffe, die schwer vollständig aus dem Wirkstoff zu entfernen sind, weisen schon in Restmengen eine unerwünschte Pflanzentoxizität auf und sollen deshalb vermieden werden. Schliesslich macht es die möglichste Vermeidung von Wasser nötig, von getrockneten, wasserfreien Verbindungen auszugehen, was deshalb von Nachteil ist, da das 3-Phenyl-4-hydroxy-6-chlorpyridazin von der Herstellung her Wasser enthält.

Überraschenderweise konnte nun gefunden werden, dass die Herstellung der Verbindungen der Formel I sehr wohl in Gegenwart von Wasser erfolgen kann, wenn sie in einem wässrig-acetonischen Lösungsmittelsystem durchgeführt wird. Dadurch kann auf die sonst nötige und aufwendige Trocknung von 3-Phenyl-4-hydroxy-6-chlorpyridazin verzichtet werden. Die Verwendung der Alkalisalze als Ausgangsmaterial war bisher deswegen nicht in Betracht gezogen worden, weil sie mit 2 Molen Kristallwasser kristallisieren, die sehr schwer durch Trocknen vollständig zu entfernen sind.

Gegenstand der vorliegenden Erfindung ist demnach ein Verfahren zur Herstellung von Phenylpyridazinverbindungen der allgemeinen Formel

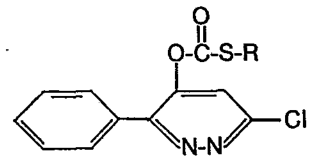

(I),

in der R einen geradkettigen oder verzweigten Alkylrest mit 1 bis 18 C-Atomen bedeutet, durch Umsetzen der Salze von 3-Phenyl-4-hydroxy-6-chlorpyridazin mit Alkylthiochlorformiaten der Formel

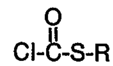

(II),

in der R wie in Formel I definiert ist, dadurch gekennzeichnet, dass als Salze die Alkalisalze verwendet werden und die Umsetzung in wässrigacetonischer Lösung bei einer Temperatur von 5 bis 60 °C erfolgt, worauf nach Umsetzung und Abtrennung der gebildeten, spezifisch schwereren, wässrigen, alkalichloridhaltigen Phase die reine Phenylpyridazinverbindung der Formel I durch Verdampfen des Acetons aus der verbliebenen acetonischen Lösung erhalten wird.

Die Stammverbindung 3-Phenyl-4-hydroxy-6-chlorpyridazin wird auf bekannte Weise durch Erhitzen von 3-Phenyl-4,6-dichlorpyridazin mit Natronlauge und anschliessendem Ansäuern und Abzentrifugieren erhalten. Als besonders vorteilhaft hat es sich erwiesen, die Stammverbindung nicht zu trocknen, sondern in noch zentrifugenfeuchtem Zustand in einem Aceton-Wassergemisch zu suspendieren und mit wässrigem Alkalihydroxyd bekannter Konzentration so lange zu versetzen, bis eine klare Lösung entstanden ist und die theoretische Menge an Alkali verbraucht ist. Dies ist bei einem pH-Wert von etwa 8,8 bis 9,7. Dadurch ist die genaue Konzentration an Stammverbindung ohne deren vorherige Trocknung und Wägung bekannt, und die für den Umsatz erforderliche Menge an Alkylthiochlorformiat ist aus dem Alkaliverbrauch leicht zu ermitteln. Als Alkalihydroxyde dienen insbesondere wässriges Natrium- oder Kaliumhydroxyd.

Die Stammverbindung kann in Wasser suspendiert und dann mit Aceton versetzt werden, sie kann in Aceton suspendiert und dann mit Wasser versetzt werden, und sie kann in einem Aceton-Wasser-Gemisch suspendiert werden. Bevorzugt soll die Aceton-Wasser-Mischung 50 bis 75 Gew.% Aceton und 25 bis 50 Gew.% Wasser enthalten wobei ein Acetongehalt von 60 bis 70 Gew.% und ein Wassergehalt von 30 bis 40 Gew.% besonders bevorzugt ist.

Es ist auch möglich, das Alkalisalz der Stammverbindung gesondert herzustellen und als solches in dem Aceton-Wasser-Gemisch zu lösen, doch ist die Erzeugung einer Lösung des Alkalisalzes in situ besonders bevorzugt.

Zu der Lösung des Alkalisalzes der Stammverbindung wird die berechnete Menge an Alkylthiochlorformiat möglichst rasch und unter

starkem Rühren zugegeben. Die Umsetzung soll bei einer Temperatur von 0 bis 60 °C, insbesondere bei einer Temperatur von 10 bis 40 °C erfolgen, da die Reaktion leicht exotherm ist, kann gegebenenfalls gekühlt werden, um innerhalb des gewünschten Temperaturbereiches zu bleiben. Nach 1 bis 2 Stunden Reaktionszeit wird das Rührwerk abgestellt, worauf leicht und ohne Zwischenschichtbildung zwei Phasen entstehen: eine spezifisch leichtere organische Phase, bestehend aus O-[3-Phenyl-6-chlorpyridazinyl-(4)]-S-(alkyl)-thiocarbonat und Aceton sowie einer spezifisch schwereren Phase, bestehend aus Wasser, Alkalichlorid und wenig Aceton. Die wässrige Phase wird abgetrennt, das darin enthaltene Aceton abdestilliert und wiederverwendet, sodass als Abwasser nur eine wässrige Kochsalz- oder Alkalichloridlösung entsteht.

Aus der organischen Phase, die nur Spuren Wasser enthält, wird das Aceton zusammen mit den Wasserspuren abdestilliert, worauf das Phenyl-chlor-pyridazinyl-alkyl-thiocarbonat in praktisch 100%iger Ausbeute zurückbleibt.

Beispiel 1:

In einem 1500-l-Rührwerkskessel wurden 550 l Aceton vorgelegt, unter Rühren 300 kg zentrifugenfeuchtes 3-Phenyl-4-hydroxy-6-chlorpyridazin dazugegeben und dann noch 220 l Wasser eingebracht. Anschliessend wurde solange 51,0%ige Natronlauge zugegeben, bis ein pH-Wert von 9,5 erreicht, und eine klare Lösung entstanden war. Es wurden 93,7 kg Lauge verbraucht. Dann wurden 254,6 kg 98%iges Octylthiochlorformiat im Laufe von 4 bis 5 Minuten bei 30 °C eingebracht. Die Temperatur steig innerhalb weniger Minuten auf 35 °C an. Durch Kühlung wurde die Reaktionstemperatur zwischen 35 und 37 °C gehalten. Während einer Stunde sank der pH-Wert auf ca. 4,5 ab. Es wurde insgesamt 2 Stunden reagieren gelassen, anschliessend wurde das Rührwerk abgestellt, 30 Minuten stehen gelassen und die spezifisch schwerere wässrige Phase abgetrennt. Von der organischen Phase wurde das Aceton rasch abdestilliert. Es wurden 456 kg techn. Produkt erhalten, entsprechend einer Ausbeute von 99,6%.

Beispiel 2:

In einem 2-l-Vierhalskolben wurde ein Gemisch von 550 ml Aceton, 320 ml Wasser, 300 g feuchtes 3-Phenyl-4-hydroxy-6-chlorpyridazin suspendiert und unter Rühren mit einer 44,7%igen wässrigen Kaliumhydroxidlösung auf pH 9,5 gestellt. Es bildete sich eine klare Lösung und es wurden 174,4 g Kalilauge verbraucht. Diese Lösung wurde auf 25 °C abgekühlt und 295 g n-Octylthiochlorformiat (98%ig) innerhalb einer Minute unter starkem Rühren zugesetzt. Im Laufe von 15 Minuten stieg die Temperatur auf 40 °C an und sank dann in den nächsten 105 Minuten wieder auf 31 °C ab.

Es wurde der Rührer abgestellt, nach 10 Minuten Absitzzeit wurden die Schichten getrennt.

Die organische Phase wurde im Rotavapor eingedampft und bei 80 °C unter Vakuum das restliche Aceton entfernt. Es wurden 536 g öliges Produkt erhalten. Ausbeute = 99,2%.

Beispiel 3:

In einem 2-l-Dreihalskolben wurden 935 ml Aceton, 374 ml Wasser und 350 g zentrifugenfeuchtes 3-Phenyl-4-hydroxy-6-chlorpyridazin suspendiert und mit 144 g 48,1%iger NaOH auf pH 9,5 gestellt.

Es wurden bei 25 °C 222 g Äthylthiochlorformiat im Laufe von 2 Minuten zugegeben. Im Verlauf von 10 Minuten stieg die Temperatur auf 39 °C an. Es wurde insgesamt 2 Stunden reagieren gelassen, dann der Rührer abgestellt und die Phasentrennung durchgeführt. Von der spezifisch leichteren organischen Phase wurde am Rotationsverdampfer unter Vakuum das Lösungsmittel total abdestilliert und 511 g öliges O-[3-Phenyl-6-chlorpyridazinyl-(4)]-S-äthylthiocarbonat erhalten. Ausbeute von 100%.

Beispiel 4:

In einem 1-l-Dreihalskolben wurden 200 ml Aceton, 100 ml Wasser und 100 g zentrifugenfeuchtes 3-Phenyl-4-hydroxy-6-chlorpyridazin vermengt und mit 39,4 g 48,1%iger NaOH auf pH 9,6 gestellt, die Lösung auf 25 °C abgekühlt und 67,0 g 98%iges Isopropylthiochlorformiat im Laufe von 30 Sekunden zugegeben.

Nach 4 Minuten war eine Temperatur von 40 °C und ein pH-Wert von 7,5 erreicht. Es wurde 2 Stunden gerührt, dabei sank der pH-Wert auf 4,5 ab. Nach dem Abstellen des Rührers erfolgte die Schichtentrennung und Abtrennung der spezifisch schweren NaCl-haltigen Phase. Die spezifisch leichtere Phase wurde am Rotationsverdampfer unter Vakuum vom Lösungsmittel befreit und 146,0 g öliges O-[3-Phenyl-6-chlorpyridazinyl-(4)]-S-isopropylthiocarbonat erhalten. Das entspricht 100% Ausbeute.

Beispiel 5:

So wie im Beispiel 4 beschrieben, wurden 100 g zentrifugenfeuchtes 3-Phenyl-4-hydroxy-6-chlorpyridazin mit 39,0 g 48,1%iger NaOH auf pH 9,6 gebracht und bei 25 °C 73 g 98%iges Isobutylthiochlorformiat zugegeben und 2 Stunden unter Rühren reagieren gelassen. Es wurde eine maximale Reaktionstemperatur von 40 °C und ein pH-Wert von 4,2 erreicht.

Nach der Phasentrennung und Abtrennung der wässrigen Phase wurde aus der spezifisch leichteren organischen Phase das Lösungsmittel verdampft. Es ergaben sich 151 g O-[3-Phenyl-6-chlorpyridazinyl-(4)]-S-isobutyl-thiocarbonat als öliges Produkt entsprechend einer Ausbeute von 100%.

**Patentansprüche**

1. Verfahren zur Herstellung von Phenylpyridazinverbindungen der allgemeinen Formel

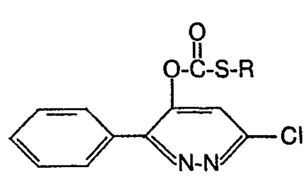

(I),

in der R einen geradkettigen oder verzweigten Alkylrest mit 1 bis 18 C-Atomen bedeutet, durch Umsetzen von Salzen des 3-Phenyl-4-hydroxy-6-chlorpyridazin mit Alkylthiochlorformiaten der Formel

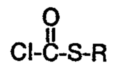 (II),

in der R wie in Formel I definiert ist, dadurch gekennzeichnet, dass als Salze die Alkalisalze verwendet werden und die Umsetzung in wässrigacetonischer Lösung bei einer Temperatur von 5 bis 60 °C erfolgt, worauf nach Umsetzung und Abtrennung der gebildeten, spezifisch schwereren, wässrigen, alkalichloridhaltigen Phase die reine Phenylpyridazinverbindung der Formel I durch Verdampfen des Acetons aus der verbliebenen acetonischen Lösung erhalten wird.

2. Verfahren nach Anspruch 1, dadurch gekennzeichnet, dass die wässrig-acetonische Lösung 50 bis 75 Gew.% Aceton und 25 bis 50 Gew.% Wasser enthält.

3. Verfahren nach Anspruch 2, dadurch gekennzeichnet, dass die wässrig-acetonische Lösung 60 bis 70 Gew.% Aceton und 30 bis 40 Gew.% Wasser enthält.

4. Verfahren nach den Ansprüchen 1 bis 3, dadurch gekennzeichnet, dass die Umsetzung bei einer Temperatur von 10 bis 40 °C erfolgt.

5. Verfahren nach den Ansprüchen 1 bis 4, dadurch gekennzeichnet, dass die Alkalisalze in situ aus 3-Phenyl-4-hydroxy-6-chlorpyridazin und einer wässrigen Alkalihydroxydlösung bekannter Konzentration gebildet werden.

**Claims**

1. Process for the preparation of phenylpyridazine compounds of the general formula

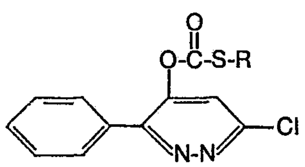 (I),

in which R is a straight-chain or branched alkyl radical having 1 to 18 carbon atoms, by reacting salts of 3-phenyl-4-hydroxy-6-chloropyridazine with alkyl thiochloroformates of the formula

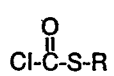 (II),

in which R is as defined in formula I, characterized in that the alkali metal salts are used as the salts and the reaction is carried out in aqueous acetone solution at a temperature of 5 to 60 °C, whereupon, after the reaction has taken place and the resulting aqueous, higher-density phase containing alkali metal chloride has been separated off, the pure phenylpyridazine compound of the formula I is obtained by evaporating the acetone from the remaining acetone solution.

2. Process according to Claim 1, characterized in that the aqueous acetone solution contains 50 to 75% by weight of acetone and 25 to 50% by weight of water.

3. Process according to Claim 2, characterized in that the aqueous acetone solution contains 60 to 70% by weight of acetone and 30 to 40% by weight of water.

4. Process according to Claims 1 to 3, characterized in that the reaction is carried out at a temperature from 10 to 40 °C.

5. Process according to Claims 1 to 4, characterized in that the alkali metal salts are formed in situ from 3-phenyl-4-hydroxy-6-chloropyridazine and an aqueous alkali metal hydroxide solution.

**Revendications**

1. Procédé pour la préparation de composés de phénylpyridazine de formule générale

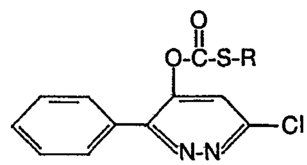 (I),

dans laquelle R représente un radical alcoyle linéaire ou ramifié ayant de 1 à 18 atomes de C, par réaction de sels de 3-phényl-4-hydroxy-6-chloropyridazine avec des thiochloroformiates d'alcoyle de formule

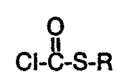 (II),

dans laquelle R est tel que défini dans la formule I, caractérisé en ce qu'on utilise comme sels les sels alcalins et en ce qu'on effectue la réaction en solution hydro-acétonique à une température de 5 à 60 °C, après quoi, après réaction et séparation de la phase aqueuse formée spécifiquement plus lourde contenant le chlorure alcalin, on abtient le composé de phénylpyridazine pur de formule I par évaporation de l'acétone à partir de la solution acétonique restante.

2. Procédé suivant la revendication 1, caractérisé en ce que la solution hydro-acétonique contient de 50 à 75% en poids d'acétone et de 25 à 50% en poids d'eau.

3. Procédé suivant la revendication 2, caractérisé en ce que la solution hydro-acétonique contient de 60 à 70% en poids d'acétone et de 30 à 40% en poids d'eau.

4. Procédé suivant les revendications 1 à 3, caractérisé en ce que la réaction a lieu à une température de 10 à 40 °C.

5. Procédé suivant les revendications 1 à 4, caractérisé en ce que les sels alcalins sont formés in situ à partir de la 3-phényl-4-hydroxy-6-chloropyridazine et d'une solution aqueuse d'hydroxyde alcalin de concentration connue.